(19) Europäisches Patentamt
European Patent Office
Office européen des brevets

(11) Numéro de publication: **0 327 417 A1**

# (12) DEMANDE DE BREVET EUROPEEN

(21) Numéro de dépôt: **89400139.5**

(22) Date de dépôt: **18.01.89**

(51) Int. Cl.4: **C 07 D 513/04**
//(C07D513/04,277:00,209:00)

(30) Priorité: **20.01.88 FR 8800591**

(43) Date de publication de la demande:
**09.08.89 Bulletin 89/32**

(84) Etats contractants désignés:
**AT BE CH DE ES FR GB GR IT LI LU NL SE**

(71) Demandeur: **RHONE-POULENC SANTE**
**20, avenue Raymond Aron**
**F-92165 Antony Cédex (FR)**

(72) Inventeur: **Rajoharison, Harivelo Gérard**
**16, allée François Villon**
**F-38130 Echirolles (FR)**

## (54) Procédé de préparation du (pyridyl-3)-3 1H,3H-pyrrolo [1,2-c] thiazolecarbonitrile-7.

(57) Procédé de préparation du nitrile de formule (I) par action du chloro-2 acrylonitrile sur l'acide formyl-3 (pyridyl-3)-2 thiazolidinecarboxylique-4 en présence d'un chlorure d'acide dans un solvant organique capable de solubiliser l'acide (II) sous forme de sel avec une base organique.

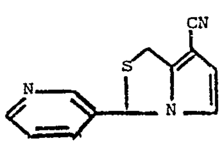

(I)

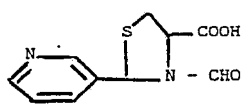

(II)

EP 0 327 417 A1

**Description**

**PROCEDE DE PREPARATION DU (PYRIDYL-3)-3 1H,3H-PYRROLO [1,2-c] THIAZOLECARBONITRILE-7**

La présente invention concerne un nouveau procédé de préparation du (pyridyl-3)-3 1H,3H-pyrrolo [1,2-c] thiazolecarbonitrile-7 de formule :

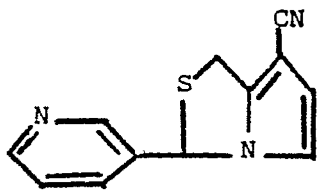

(I)

utile comme intermédiaire dans la préparation de médicaments destinés à traiter les affections dans lesquelles est impliqué le rôle physiologique du PAF-acéther, notamment pour soigner les allergies, l'inflammation et empêcher l'agrégation des plaquettes de sang. De tels produits sont connus par le brevet européen n⁰ 0 115 979.

Jusqu'à ce jour, le (pyridyl-3)-3 1H,3H pyrrolo [1,2-c] thiazolecarbonitrile-7 était préparé selon le procédé décrit dans le brevet européen n⁰ 0 115 979 à partir de l'acide formyl-3 (pyridyl-3)-2 thiazolidinecarboxylique-4 selon le schéma suivant :

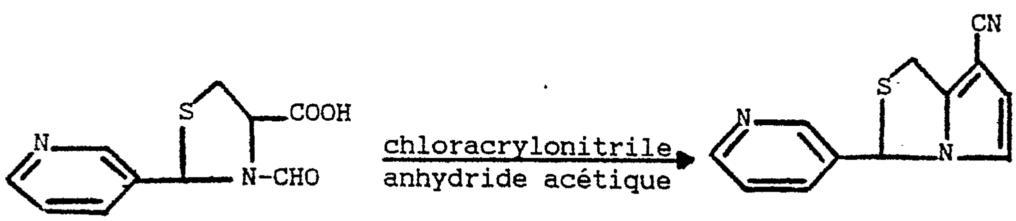


(II) (III)

Cette synthèse présente deux inconvénients principaux :
- Quelle que soit la pureté du produit formylé de formule (II) mis en oeuvre, on obtient toujours des quantités importantes de goudrons, ce qui nécessite des opérations délicates de purification et des rendements faibles (de l'ordre de 15%).
- La condensation de chloracrylonitrile sur l'acide de formule (II) nécessite 5 équivalents de chloracrylonitrile par mole d'acide, ce qui entraîne l'obligation de récupérer et de recycler le réactif lorsqu'on envisage un procédé à l'échelle industrielle.

Il a maintenant été trouvé, et c'est ce qui fait l'objet de la présente invention, que ces inconvénients pouvaient être évités en remplaçant l'anhydride acétique par un chlorure d'acide, en employant un solvant à bas point d'ébullition permettant d'effectuer la réaction dans des conditions douces et en solubilisant l'acide de formule (II) dans le solvant sous forme d'un sel avec une base organique.

Dans la pratique, on effectue généralement la réaction dans un solvant organique chloré capable de dissoudre le sel de l'acide de formule (II), tel que le dichlorométhane ou le dichloroéthane en opérant à une température comprise entre - 65°C et la température de reflux du mélange réactionnel en présence du chlorure d'acide choisi.

Comme base organique pour la formation du sel de l'acide de formule (II), on peut employer une trialcoylamine telle que la triéthylamine, la tributylamine, ou la pyridine.

Comme chlorure d'acide, il est particulièrement avantageux, sans que cela soit limitatif, d'employer le chlorure de paratoluène sulfonyle, le chlorure de méthanesulfonyle, le chlorure d'oxalyle ou l'oxychlorure de phosphore.

Le nitrile de formule (I) peut être purifié par les moyens habituels connus de l'homme du métier, par exemple par chromatographie ou recristallisation.

L'acide de formule (II) peut être préparé selon la méthode décrite dans le brevet européen 0 115 979.

Les exemples suivants montrent de façon plus détaillée, comment dans la pratique l'invention peut être réalisée.

EXEMPLE 1

A une suspension de 4,47 g d'acide formyl-3 (pyridyl-3)-2 thiazolidinecarboxylique-4 dans 40 cm3 de dichlorométhane, on ajoute 7,67 g de triéthylamine à une température voisine de 20° C. On obtient une solution limpide jaune clair. On refroidit le mélange à -65° C puis on ajoute goutte à goutte en 10 minutes 1,84 g d'oxychlorure de phosphore. La suspension initiale fait place à un mélange homogène de couleur rouge voliacé. On laisse agiter pendant 1 heure à -65° C puis on ajoute rapidement en 4 minutes 4,38 g de chloro-2 acrylonitrile. On laisse revenir progressivement en 1 heure le mélange réactionnel à une température voisine de 20° C puis on ajoute 20 cm3 de dichlorométhane et chauffe à 40° C pendant 1 heure. Après refroidissement à température ambiante, on ajoute 20 cm3 d'eau et 10 cm3 d'une solution aqueuse de soude 0,5N. On décante la phase organique et extrait plusieurs fois la phase aqueuse par du dichlorométhane. Les extraits organiques sont rassemblés et séchés sur du sulfate de sodium pendant une nuit. Après filtration, le solvant est évaporé sous pression réduite (20 mm de mercure; 2,7 kPa) à 40° C; on obtient 2,95 g de produit brut. Ce produit est chromatographié sur une colonne de 25 mm de diamètre contenant 50 g de silice (0,063-0,2 mm) en éluant avec de l'acétate d'éthyle. On obtient ainsi 1,84 g de cyano-7 (pyridyl-3)-3 1H,3H-pyrrolo [1,2-c] thiazole sous forme de cristaux de couleur crème fondant à 110° C (rendement 40% par rapport à l'acide engagé).

EXEMPLE 2

A une suspension agitée de 4,77 g d'acide formyl-3 (pyridyl-3)-2 thiazolidinecarboxylique-4 dans 30 cm3 de dichloro-1,2 éthane en présence de quelques cristaux d'hydroquinone, on ajoute goutte à goutte en 15 minutes 3,1 cm3 de triéthylamine à une température voisine de 20° C. On obtient un mélange homogène de couleur jaune clair. On ajoute ensuite en 1 heure et 10 minutes à une température voisine de 20° C une solution de 2,76 g de chlorure d'oxalyle dans 10 cm3 de dichloro-1,2 éthane. Le mélange réactionnel devient hétérogène de couleur ocre. On ajoute alors en 20 minutes à 20° C une solution contenant 2 g de chloro-2 acrylonitrile, 6,15 cm3 de triéthylamine et 10 cm3 de dichloro-1,2 éthane. On laisse agiter pendant 1 heure à une température voisine de 20° C puis on chauffe à 40° C pendant 1 heure. Après refroidissement, on ajoute 75 cm3 d'eau distillée. La phase organique est décantée, séchée sur du sulfate de sodium puis dosée en CLHP. Elle contient 2,51 g de cyano-7 (pyridyl-3)-3 1H,3H-pyrrolo [1,2-c] thiazole, soit un rendement de 55% par rapport à l'acide engagé.

EXEMPLE 3

Dans un tricol de 50 cm3 muni d'un réfrigérant, d'une ampoule de coulée, d'une agitation magnétique, d'un thermomètre et d'un gazomètre, on met en suspension 10 g d'acide formyl-3 (pyridyl-3)-2 thiazolidinecarboxylique-4 dans 15 cm3 de dichlorométhane. On ajoute ensuite 4,8 g de triéthylamine à 20° C. On obtient une solution homogène incolore. Cette solution est ajoutée goutte à goutte en 50 minutes et à 25° C à un mélange agité de 5,29 g de chlorure de méthanesulfonyle et 17 cm3 de dichlorométhane. On ajoute ensuite rapidement 4,04 g de chloro-2 acrylonitrile puis, en une heure à 20-35° C, 9,35 g de triéthylamine et 5 cm3 de dichlorométhane. On porte ensuite le mélange à reflux pendant 50 minutes. Après refroidissement, on hydrolyse par 12 cm3 d'eau. On décante la phase organique et extrait la phase aqueuse par du dichlorométhane. Les extraits organiques sont rassemblés et séchés sur du sulfate de sodium pendant une nuit. Après filtration, le solvant est évaporé sous pression réduite (20 mm de mercure; 2,7 kPa) à 20° C. On obtient 9 g de produit brut que l'on purifie par chromatographie sur une colonne de 4,5 cm de diamètre contenant 100 g de silice (0,063-0,2 mm) en éluant avec un mélange d'acétate d'éthyle et de dichlorométhane. On obtient ainsi 7,10 g de cyano-7 (pyridyl-3)-3 1H,3H-pyrrolo [1,2-c] thiazole sous forme de cristaux de couleur beige clair fondant à 108° C (rendement 82,5% par rapport à l'acide engagé).

EXEMPLE 4

A une suspension 10 g d'acide formyl-3 (pyridyl-3)-2 thiazolidinecarboxylique-4 dans 15 cm3 de dichlorométhane, on ajoute 4,67 g de triéthylamine à une température voisine de 20° C. On obtient une solution limpide jaune clair. Cette solution est ajoutée goutte à goutte en 1 heure à 40° C à un mélange de 8,8 g de chlorure de tosyle dans 17 cm3 de dichlorométhane. Le mélange réactionnel devient homogène de couleur rouge orangé. On ajoute alors rapidement 4,10 g de chloro-2 acrylonitrile, puis, en maintenant le reflux du milieu réactionnel, 9,37 g de triéthylamine. On maintient le mélange réactionnel à reflux pendant encore 1 heure et 25 minutes. Après refroidissement à température ambiante, on hydrolyse le milieu réactionnel avec 12 cm3 d'eau. On décante la phase organique et extrait la phase aqueuse par du dichlorométhane. Les extraits organiques sont rassemblés et séchés sur du sulfate de sodium pendant une nuit. Après filtration, le solvant est évaporé sous pression réduite (20 mm de mercure; 2,7 kPa) à 20° C. On obtient 11,11 g de produit brut que l'on purifie par chromatographie sur une colonne de 4,5 cm de diamètre contenant 100 g de silice (0,063-0,2 mm) en éluant avec un mélange d'acétate d'éthyle et de dichlorométhane. On obtient ainsi 8,36 g de cyano-7 (pyridyl-3)-3 1H,3H-pyrrolo [1,2-c] thiazole sous forme de cristaux de couleur beige clair clair fondant à 110° C (rendement 88,4% par rapport à l'acide engagé).

**Revendications**

1 - Procédé de préparation du (pyridyl-3)-3 1H-3H-pyrrolo [1,2-c] thiazolecarbonitrile-7 de formule :

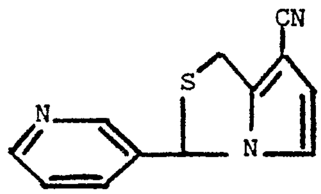

(I)

par action du chloracrylonitrile sur l'acide formyl-3 (pyridyl-3)-2 thiazolidinecarboxylique-4 de formule :

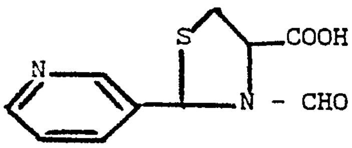

(II)

caractérisé en ce que l'on opère en présence d'un chlorure d'acide dans un solvant organique capable de solubiliser l'acide de formule (II) sous forme de sel avec une base organique.

2 - Procédé selon la revendication 1, caractérisé en ce que le chlorure d'acide employé est choisi parmi le chlorure de paratoluène-sulfonyle, le chlorure de méthanesulfonyle, le chlorure d'oxalyle ou l'oxychlorure de phosphore.

3 - Procédé selon la revendication 1, caractérisé en ce que la base utilisée pour former le sel avec l'acide de formule (II) est une trialcoylamine ou la pyridine.

4 - Procédé selon la revendication 3, caractérisé en ce que la trialcoylamine est la triéthylamine.

5 - Procédé selon la revendication 1, caractérisé en ce que le solvant utilisé est un solvant chloré.

6 - Procédé selon la revendication 5, caractérisé en ce que le solvant chloré est le dichlorométhane ou le dichloro-1,2 éthane.

Office européen des brevets

# RAPPORT DE RECHERCHE EUROPEENNE

Numero de la demande

EP 89 40 0139

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,A | EP-A-0 115 979 (RHONE-POULENC SANTE)<br>* revendication 2; exemple 1 * | 1 | C 07 D 513/04 //<br>(C 07 D 513/04<br>C 07 D 277:00<br>C 07 D 209:00 ) |
| A | EP-A-0 252 823 (RHONE-POULENC SANTE)<br>* revendication 3 * | 1-4 | |

| DOMAINES TECHNIQUES RECHERCHES (Int. Cl.4) |
|---|
| C 07 D 277/00<br>C 07 D 513/00 |

Le présent rapport a été établi pour toutes les revendications

| Lieu de la recherche | Date d'achèvement de la recherche | Examinateur |
|---|---|---|
| BERLIN | 03-04-1989 | HASS C V F |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)